# EUROPEAN PATENT APPLICATION

(11) **EP 3 026 105 A1**
(43) Date of publication of application: **01.06.2016**
(21) Application number: 14004016.3
(22) Date of filing: 27.11.2014
(51) Int. Cl.: C12M 1/00

(54) **Silicone hose for solving CO2 in water for algae production**

(71) Applicant: Linde Aktiengesellschaft, 80331 München (DE)
(72) Inventor: van de Ven, Joost, 5235 DC 's-Hertogenbosch (NL)
(74) Representative: Gellner, Bernd

(57) **Abstract**

The invention relates to a method for discharging CO₂ into a liquid (5) comprising water and algae, wherein gaseous CO₂ is discharged via a conduit (1) into said liquid (5). According to the invention, gaseous CO₂ is diffused through a closed wall (2) of said conduit (1) into the liquid (5), wherein said wall (3) comprises a polysiloxane or is formed out of at least one polysiloxane. Furthermore, the invention relates to a corresponding system.

## Description

The invention relates to a method for growing algae, wherein CO₂ is discharged into a liquid comprising water and algae according to claim 1 as well as to a corresponding system according to claim 5.

For growing algae CO₂ has to be added to have an increased growth of algae.

Algae are grown for nutrients, feed for fish and other animals, cosmetics, base products for medicine production, bio plastics, omega 3-6, and for the production of biodiesel and many other products. In principle, besides algae, the invention can be applied to all autotrophic CO₂-assimilating organisms.

For a high production rate of the algae one needs:
- Light, typically sun light,
- Nutrients,
- Water (e.g. fresh, brackish or salt water),
- CO₂, and particularly
- Mixing (circulation).

When using pure CO₂ in algae farming the efficiency of solving the CO₂ is very important. Most systems use perforated hoses or plates to solve the CO₂. The water depth in algae farms is typically relatively low (e.g. 15 to 20 cm).

The efficiency of the CO₂ dosing on those depths is not high (30% to 90% depending on type of injection system and algae species). Often sumps are used to have a higher water depth and efficiency of the dosed CO₂. This increases the costs of the algae pond.

Further, a sump - from a hydrological point of view - is not a very good technical solution, since the flow speed in the sump normally decreases significantly so that the CO₂ will build up and lower the efficiency of the CO₂ exchange, and sedimentation of algae and other materials can occur.

Based on the above, the problem underlying the present invention is to provide a method/system of the afore-mentioned kind that improves the efficiency of solving the CO₂ in the liquid (e.g. water with algae).

This problem is solved by a method having the features of claim 1. Preferred embodiments are stated in the corresponding sub claims.

According to claim 1, gaseous CO₂ is diffused through a closed wall of said conduit into the liquid, wherein said wall of the conduit comprises a polysiloxane or is formed out of at least one polysiloxane.

Polysiloxanes (also denoted as silicones) are polymers that include silicon together with carbon, hydrogen, oxygen, and sometimes other elements.

Silicones are mixed inorganic-organic polymers having the formula [R₂SiO]ₙ, where R is an organic group (e.g. methyl, ethyl, or phenyl). Silicones comprise an inorganic
silicon-oxygen backbone (···-Si-O-Si-O-Si-O-···) with organic side groups attached to
the silicon atoms, which are four-coordinate.

Furthermore, organic side groups may be used to link two or more of said -Si-O-backbones together. By varying the -Si-O- chain lengths, side groups, and crosslinking, silicones can be synthesized with a wide variety of properties and compositions. The most common siloxane, which is preferably used in the present invention, is linear polydimethylsiloxane (PDMS).

Therefore, according to a preferred embodiment of the present invention said polysiloxane is PDMS.

Further, preferably said conduit is a hose, particularly a flexible hose, wherein said wall particularly is a circumferential wall of said hose.

Further, preferably, the gaseous CO₂ is moistened with H₂O before diffusing it through the wall of the conduit into the liquid.

Preferably, the pressure of the CO₂ in the conduit lies within the range from 0.2 bar(o) to 5bar(o), preferably in the range between 0.2 bar(o) and 4 bar(o). Preferably, the temperature of the water lies within the range from 10°C to 35°C.

Further, the problem underlying the present invention is solved by a system having the features of claim 5. Preferred embodiments are stated in the corresponding sub claims.

According to claim 5, said conduit being designed to reside in the liquid comprises a closed wall designed for diffusing gaseous CO₂ through said wall and into said surrounding liquid, wherein said wall comprises a polysiloxane or is formed out of at least one polysiloxane.

Preferably, said conduit is formed as a hose, particularly a flexible hose, wherein said wall particularly is a circumferential wall of said hose.

In the following, further features, advantages and examples of the present invention shall be described with reference to the Figures, wherein
- Fig. 1: shows a schematical cross sectional view of a silicone hose for dosing CO₂ into water of an algae pond,
- Fig. 2: shows pH versus time upon discharging CO₂ via a silicone hose into water, and
- Fig. 3: shows pH versus time upon discharging CO₂ via a silicone hose into water containing algae.

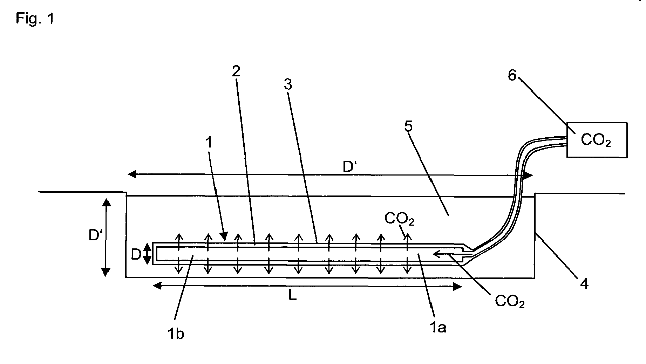

Fig. 1 shows a container or pond 4 of diameter D filled with water 5 of depth D' for algae farming. In order to discharge CO₂ into the water 5, a hose 1 comprising a circumferential closed wall 2 (i.e. without apertures formed into the wall) out of silicone having an outer surface 3. CO₂ is fed into an interior space defined by said wall 2 of the hose 1 and diffuses through the silicone wall 2 of the hose 1 into the water 5.

The hose 1 has a length L along a longitudinal extension direction and a diameter D" as well as a wall thickness T.

### Example 1

In a first example CO₂ was discharged through the silicone hose 1 into drinking water 5 comprising NaOH residing in a tank 4 having a diameter D of 28cm. The water depth was D'=15.5 cm. The pH of said mixture amounted to 10.99 initially.

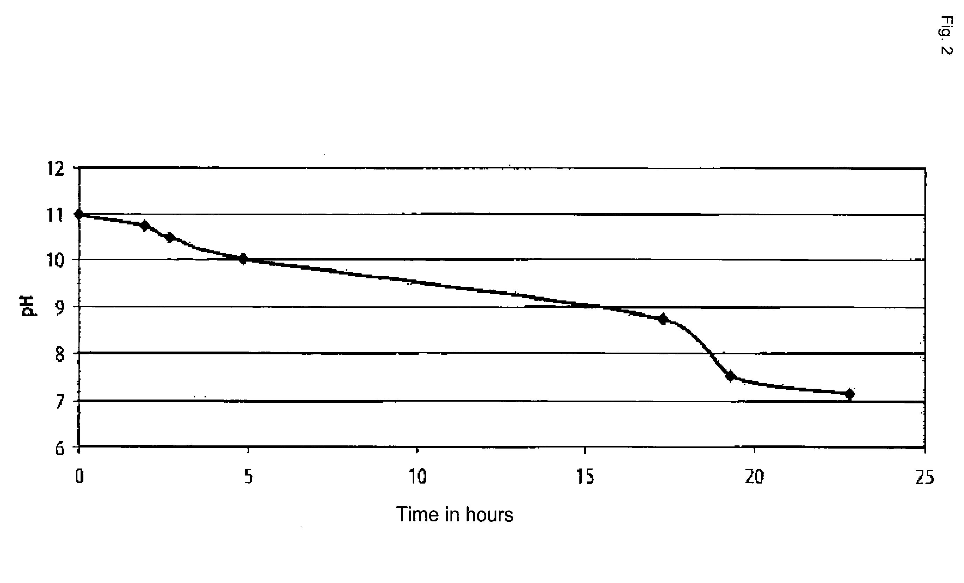

As shown in Fig. 2, upon discharging CO₂ via the silicone hose 1 into the water 5, the pH dropped with increasing time.

### Example 2

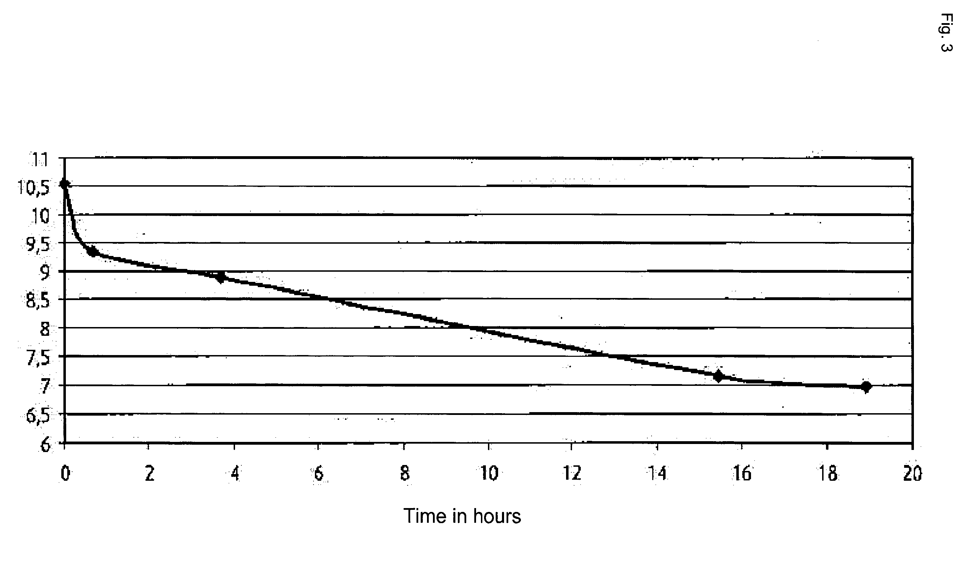

In a second example CO₂ was discharged through said silicone hose 1 into water 5 containing algae. At first, CO₂ dosing was stopped until the pH of the water increased and then CO₂ dosing started again. Also here, the pH dropped over time as shown in Fig. 3.

Both tests were performed with a silicone hose 1 having a diameter D"=8mm, a length L= 48 cm and a wall thickness T=1.5 mm. The CO₂ pressure in the hose 1 during the tests was 1.5 bar(o).The temperature of the water was 25°C. The silicone used was PDMS.

On the outside of the hose 1 bubbles were formed, presumably due to local oversaturation of gases. The bubbles stayed a relatively long time, e.g. within the range from 3 min to 5 min, on the hose's 1 surface 3 before getting up to the surface of the water 5 in the tank 4.

When releasing the bubbles from the hose 1 by moving the hose 1, every time new bubbles formed. Presumably, CO₂ is contained in said bubbles, which may possibly react to form bicarbonates and carbonates especially at higher pH.

The hose 1 resided in the water 5 with algae for three weeks and no scaling or fouling on the hose 1 occurred.

The surface 3 of the hose 1 probably has a low pH due to the CO₂ and the bubbles formed all over the surface 3 of the hose 1 prevent fouling.

Furthermore, it was observed that when dosing pure CO₂ in a first part 1a of the hose 1, less transport of CO₂ through the hose wall occurred than in the second part 1b of the hose.

This can be related to moisture diffusing through the silicone wall 2 of the hose 1 which helps the transport of CO₂ through the hose wall 2. When the CO₂ enters the silicone hose 1 it has no moisture, but the hose 1 itself allows water to diffuse through the hose 1 and get into the interior space of the hose 1. So, in the beginning of the hose 1 the CO₂ is slowly moistened. As the CO₂ gas gets more moistened the transport through the hose 1 gets better, which can be noticed because there are more bubbles on the respective part on the outside of the hose 1.

Preferably, CO₂ is moistened with water by leading the gas through a water columm before dosing it into the silicone hose 1 to get a better efficiency of the CO₂ transport through the hose wall 2. Other ways are also conceivable.

**Reference Numerals**

| | |
|---|---|
| 1 | conduit or hose |
| 2 | Wall |
| 3 | Surface |
| 4 | Container, tank or pond |
| 5 | Liquid (e.g. water with algae) |
| 6 | CO₂ source |
| D, D' | Diameter |
| D" | Depth |
| T | Thickness |
| L | Length |

## Claims

1. Method for growing algae, wherein CO₂ is discharged into a liquid (5) comprising water and algae so that CO₂ is assimilated by said algae, wherein gaseous CO₂ is discharged via a conduit (1) residing in said liquid (5) into said liquid (5),
wherein
gaseous CO₂ is diffused through a closed wall (2) of said conduit (1) into the liquid (5), wherein said wall (3) comprises a polysiloxane or is formed out of at least one polysiloxane.

2. Method according to claim 1, **characterized in that** said polysiloxane is Polydimethylsiloxane (PDMS).

3. Method according to claim 1 or 2, **characterized in that** said conduit (1) is a hose, wherein said wall (2) particularly is a circumferential wall (2) of said hose (1).

4. Method according to one of the preceding claims, **characterized in that** the gaseous CO₂ is moistened with H₂O before diffusing it through the wall (2) of the conduit (1) into the liquid (5).

5. System for growing algae, comprising:
- a container (4) containing a liquid comprising water and algae,
- a conduit (1) for discharging gaseous CO₂ into the liquid (5), and
- a CO₂ source (6) designed to be in flow connection with said conduit (1),
wherein
said conduit (1) comprises a closed wall (2) designed for diffusing gaseous CO₂ through said wall (2) and into said liquid (5), wherein said wall (2) comprises a polysiloxane or is formed out of at least one polysiloxane.

6. System according to claim 5, **characterized in that** said polysiloxane is Polydimethylsiloxane (PDMS).

7. System according to claim 5 or 6, **characterized in that** said conduit (1) is a hose, wherein said wall (2) particularly is a circumferential wall (2) of said hose (1).
